# EUROPEAN PATENT SPECIFICATION

(11) **EP 3 524 062 B1**
(45) Date of publication and mention of the grant of the patent: **21.08.2024**
(21) Application number: 19163640.6
(22) Date of filing: 14.06.2016
(51) Int. Cl.: A23L 27/20

(54) **CYCLOHEXANECARBOXYLIC ACIDS FOR SELECTIVE TASTE MASKING**
CYCLOHEXANCARBONSÄUREN ZUR SELEKTIVEN GESCHMACKSMASKIERUNG
ACIDES CYCLOHEXANECARBOXYLIQUES POUR LE MASQUAGE SÉLECTIF DU GOÛT

(30) Priority: 22.06.2015 US 201514746251
(43) Date of publication of application: 14.08.2019
(62) Divisional of application: 16815058.9
(73) Proprietor: International Flavors & Fragrances Inc., New York, NY 10019 (US)
(72) Inventor: SINGH, Haribansh Kumar, Eatontown, NJ New Jersey 07724 (US); JOHN, Thumpalasseril V., Morganville, NJ New Jersey 07751 (US); KIM, Jung-A, Edgewater, NJ New Jersey 07020 (US); RENNIE, Laura, New Brunswick, NJ New Jersey 08901 (US); SINGH, Ajay Pratap, Highland Park, NJ New Jersey 08904 (US); MURANO, Kathryn, Staten Island, NY New York 10314 (US)
(74) Representative: Barker Brettell LLP

(56) References cited:
- JP-A- 2006 006 318
- US-A1- 2012 189 750
- US-A1- 2014 045 937
- CLIFFORD M N: "CHLOROGENIC ACIDS AND ORTHER CINNAMATES - NATURE, OCCURRENCE, DIETARY BURDEN, ABSORPTION AND METABOLISM", JOURNAL OF THE SCIENCE OF FOOD AND AGRICULTURE, WILEY & SONS, CHICHESTER, GB, vol. 80, no. 7, 15 May 2000 (2000-05-15), pages 1033 - 1043, XP000927233, ISSN: 0022-5142, DOI: 10.1002/(SICI)1097-0010(20000515)80:7<1033::AID-JSFA595>3.0.CO;2-T
- JOON-KWAN MOON ET AL: "Role of Roasting Conditions in the Level of Chlorogenic Acid Content in Coffee Beans: Correlation with Coffee Acidity", JOURNAL OF AGRICULTURAL AND FOOD CHEMISTRY, vol. 57, no. 12, 24 June 2009 (2009-06-24), US, pages 5365 - 5369, XP055371741, ISSN: 0021-8561, DOI: 10.1021/jf900012b

## Description

### Field of the Invention

The present invention relates to novel compositions and uses thereof for selective masking of sour taste in consumables.

### Background of the invention

Food industry has made significant effort to modify and harmonize taste as well as to eliminate and reduce off-taste in food products. The basic categories of taste are salt}', sweet, sour, bitter and umami. Additional taste may also include, for example, metallic, burning, pungent, astringent and many more. Accordingly, the categories of off-taste are very broad and include any unwanted taste. The discovery of new taste modifiers that mask unpleasant off -taste enables the creation of well-balanced flavors. However, currently available taste modifiers are generally non-selective that the quality of the taste as a whole may be changed. Thus, it is of particular need to develop taste modifiers that provide off-taste specific masking effect. Such compounds selectively mask a particular type of off-taste without impacting the other flavors in a product.

US Patent Application 2012/0189750 relates to the use of chlorogenic acid as an agent for masking an off-taste such as bitterness.

JP Patent 2006 006318 describes food compositions containing a taste-modifying agent.

### Summary of the invention

This invention provides novel cyclohexanecarboxylic acid compositions and uses thereof in selective masking of sour taste in consumables.

In one embodiment, the present invention provides a combination consisting essentially of an olfactory effective amount of (1S,3R,4R,SR)-3-[[3-(3,4-dihydroxyphenyl)-1-oxo-2-propen-1-yl]oxy]-1,4,5-trihydroxy-cyclohexanecarboxylic acid and a consumable acid, wherein the olfactory effective amount is from 1 part per billion to 1000 parts per million, and wherein (1S,3R,4R,5R)-3-[[3-(3,4-dihydroxyphenyl)-1-oxo-2-propen-1-yl]oxy] -1,4,5-trihydroxy-cyclohexanecarboxylic acid provides a sour taste-masking effect in the consumable acid.

In another embodiment, the present invention is directed to use of an olfactory effective amount of (1S,3R,4R,5R)-3-[[3-(3,4-dihydroxyphenyl)-1-oxo-2-propen-1-yl]oxy]-1,4,5-trihydroxy-cyclohexanecarboxylic acid to mask the sour taste of a consumable, wherein the olfactory effective amount is from 1 part per billion to 1000 parts per million.

### Detailed Description of the Invention

The intensity of a sour taste in a consumable is determined by both free hydrogen ions and undissociated hydrogen ions, which are approximately equal in sour taste on a molar basis (Li, et al. Food Chem. 2005, 185:200-204). Hosokawa, *et al.* use amino acids including proline, aspartic acid and glutamic acid to modify the sourness of a citrus fruit drink but there remain problems such as unpleasant aftertaste, discolored products as well as unpleasant smell (JP 58- 138363). Washino *et al.* further teach the enhancement of sourness using a flavone derivative (EP 0605261).

In other taste areas such as sweet taste, Lee et al. teach the use of derivatives of chlorogenic acid, cynarin, isochlorogenic acid and neocholorenic acid as sweetness inducers (US 3,924,017 and US 3,916,028). Chien *et al.* later report that a chlorogenic acid mixture of 3-CQA, 4-CQA, 5-CQA, 3-FQA, 4-FQA, 5-FQA, 3-p-CoQA, 4-p CoQA, 5-p-CoQA, 3,4-diCQA, 3,5-diCQA, 4,5-diCQA, 3,4-CFQA, 3,5-CFQA and 4,5-CFQA can also be used to reduce multiple types of off-taste including the metallic and/or bitter flavors in artificial sweeteners, the burning taste in alcohol, the beany and aldehyde-like taste in soy products as well as the burning sensation in carbonated products. It is thought that such general effect may be a consequence of overall enhancement of sweetness perception (WO 02/100192).

However, nothing in these prior art provides a sour or bitter taste specific masking composition.

(1S,3R,4R,5R)-3-[[3-(3,4-Dihydroxyphenyl)-1-oxo-2-propen-1-yl]oxy]-1,4,5-trihydroxy-cyclohexanecarboxylic acid (Formula I) contains chiral centers, thereby providing a number of isomers such as (1R,3R,4S,5R)-3-[[(2E)-3-(3,4-dihydroxyphenyl)-1-oxo-2-propen-1-yl]oxy] -1,4,5-trihydroxy-cyclohexanecarboxylic acid (Formula II). (1S,3R,4R,SR)-3-[[3-(3,4- Dihydroxyphenyl)-1-oxo-2-propen-1-yl]oxy]-1,4,5-trihydroxy-cyclohexanecarboxylic acid also has a number of known close analogs such as (1α,3R,4α,5R)-4-[[3-(3,4-dihydroxyphenyl)-1-oxo-2-propen-1-yl]oxy]-1,3,5-trihydroxy-cyclohexanecarboxylic acid (Formula III), (1S,3R,4R,5R)-3, 4-bis[[3-(3,4-dihydroxyphenyl)-1-oxo-2-propenyl]oxy]-1,5-dihydroxy-cyclohexanecarboxylic acid (Formula IV), (1α,3R,4α,5R)-3,5-bis[[3-(3,4-dihydroxyphenyl)-1-oxo-2-propen-1-yl]oxy]-1,4-dihydroxy-cyclohexanecarboxylic acid (Formula V) and (1R,3R,4S,5R)-3,4-bis[[3-(3,4-dihydroxyphenyl)-1-oxo-2-propen-1-yl]oxy]-1,5-dihydroxy-cyclohexanecarboxylic acid (Formula VI). The cyclohexanecarboxylic acids of the present disclsoure are represented by formulas set forth in the following.

### (1S,3R,4R,SR)-3-[[3-(3,4-dihydroxyphenyl)-1-oxo-2-propen-1-yl]oxy]-1,4,5-trihydroxy- cyclohexanecarboxylic acid:

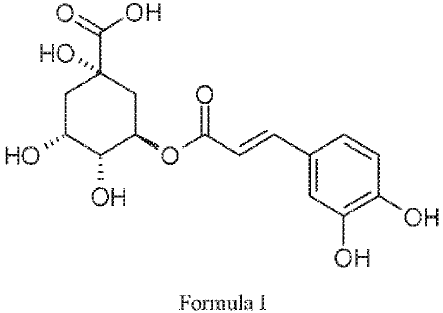

### (1R,3R,4S,5R)-3-[[(2E)-3-(3,4-dihydroxyphenyl)-1-oxo-2-propen-1-yl]oxy]-1,4,5-trihydroxy-cyclohexanecarboxylic acid:

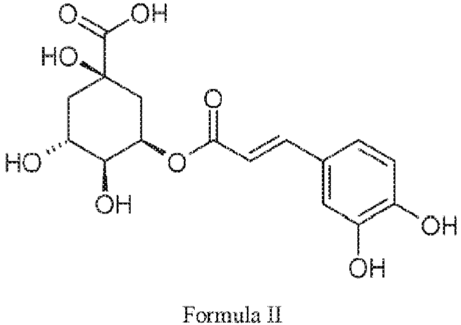

### (1α,3R,4α,5R)-4-[[3-(3,4-dihydroxyphenyl)-1-oxo-2-propen-1-yl]oxy]-1,3,5-trihydroxy-cyclohexanecarboxylic acid:

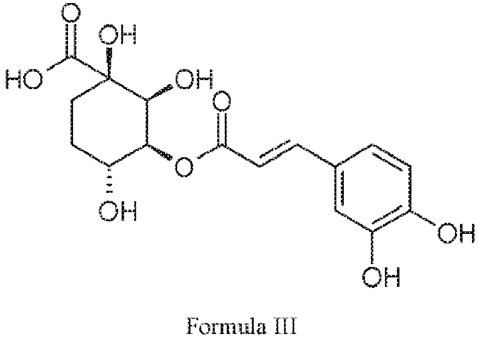

### (1S,3R,4R,5R)-3,4-bis[[3-(3,4-dihydroxyphenyl)-1-oxo-2-propenyl]oxy]-1,5-dihydroxy-cyclohexanecarboxylic acid:

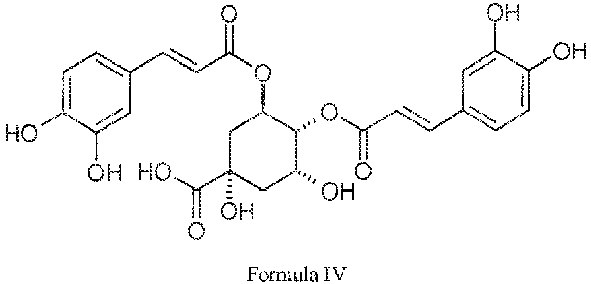

### (1α,3R,4α,5R)-3,5-bis[[3-(3,4-dihydroxyphenyl)-1-oxo-2-propen-1-yl]oxy]-1,4-dihydroxy- cyclohexanecarboxylic acid:

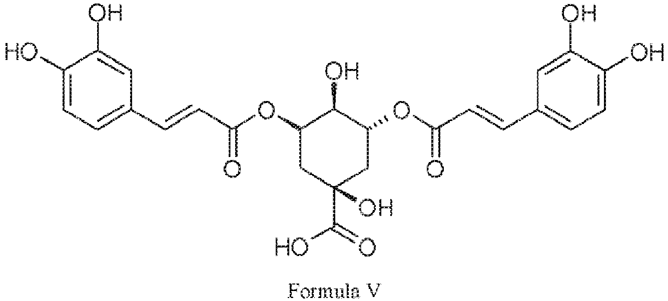

### (1R,3R,4S,SR)-3,4-bis[[3-(3,4-dihydroxyphenyl)-1-oxo-2-propen-1-yl]oxy]-1,5-dihydroxy-cyclohexanecarboxylic acid:

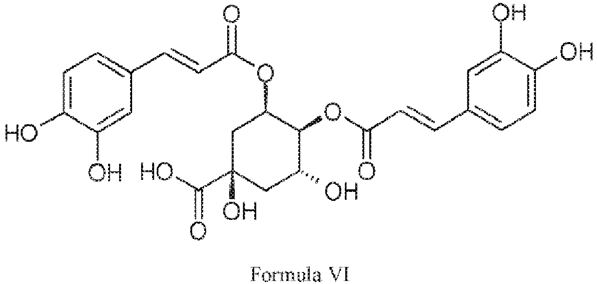

The cyclohexanecarboxylic acids of the present disclosure can be obtained commercially, synthesized according to procedures known in the art, for example, as described by Sefkow (Eur. J. Org. Chem. 2001, 1137-1141) or obtained from a variety of botanicals such as fruits (for example, apples, apricots, blackberries, blueberries, cherries, citrus fruits, peaches, pears, plums and strawberries), plant leaves (for example, blueberry mate and eucommia leaves), vegetables (for example, artichokes, Brussels sprouts, cabbages, carrots, eggplants, kales, peppers, potatoes and tomatoes) and other plants (for example, bamboos, coffee beans, honeysuckle flowers, sunflower seeds and yerba mate).

Those with skill in the art appreciate how small structural differences between close analogs can result in unexpected and significant differences in properties and functions. Further, the activities of many compounds may also associate with their chiral configuration. A compound of a wrong enantiomeric form may lack desirable biological, physical or chemical properties. However, identifying a chiral center and developing a cost-effective process to synthesize enantiomers and/or targeted racemic compounds pose difficult challenges, let alone discovering an active form is unpredictable as such effort may often not lead to a desirable enantiomer that possesses stronger function than the others and/or its racemate.

To date, there is no disclosure in the art of the flavor use associated with individual cyclohexanecarboxylic acids, let alone their selective masking of taste in consumables.

It has now been surprisingly discovered that the cyclohexanecarboxylic acids of the present disclosure possess taste specific masking effect. Particularly, (1S,3R,4R,5R)-3-[[3-(3,4-dihydroxyphenyl)-1-oxo-2-propen-1-yl]oxy]-1,4,5-trihydroxy-cyclohexanecarboxylic acid is distinctly effective in masking sour taste and (1S,3R,4R,5R)-3,4-bis[[3-(3,4-dihydroxyphenyl)-1-oxo-2-propenyl]oxy]-1,5-dihydroxy-cyclohexanecarboxylic acid,(1α,3R,4α,5R)-3,5-bis[[3-(3,4-dihydroxyphenyl)-1-oxo-2-propen-1-yl]oxy]-1,4-dihydroxy-cyclohexanecarboxylic acid and (1R,3R,4S,SR)-3,4-bis[[3-(3,4-dihydroxyphenyl)-1-oxo-2-propen-1-yl]oxy]-1,5-dihydroxy- cyclohexanecarboxylic acid are distinctly effective in masking bitter taste. Thus, (1S,3R,4R,5R)- 3-[[3-(3,4-dihydroxyphenyl)-1-oxo-2-propen-1-yl]oxy]-1,4,5-trihydroxy-cyclohexanecarboxylic acid, (1S,3R,4R,5R)-3,4-bis[[3-(3,4-dihydroxyphenyl)-1-oxo-2-propenyl]oxy]-1,5-dihydroxy-cyclohexanecarboxylic acid, (1α,3R,4α,5R)-3,5-bis[[3-(3,4-dihydroxyphenyl)-1-oxo-2-propen-1-yl]oxy]-1,4-dihydroxy-cyclohexanecarboxylic acid and (1R,3R,4S,5R)-3,4-bis[[3-(3,4-dihydroxyphenyl)-1-oxo-2-propen-1-yl]oxy]-1,5-dihydroxy-cyclohexanecarboxylic acid provide superior ingredient performance and possess unexpected advantages in specific taste masking applications.

Accordingly, one embodiment of the present invention relates to the surprising finding of the unexpected effectiveness of (1S,3R,4R,5R)-3-[[3-(3,4-dihydroxyphenyl)-1-oxo-2-propen-1-yl]oxy]-1,4,5-trihydroxy-cyclohexanecarboxylic acid in masking the sour taste of a consumable. Another surprising finding disclosed herein, but not part of the claimed invention, is the unexpected effectiveness of (1S,3R,4R,5R)-3,4-bis[[3-(3,4-dihydroxyphenyl)-1-oxo-2-propenyl]oxy]-1,5- dihydroxy-cyclohexanecarboxylic acid, (1α,3R,4α,5R)-3,5-bis[[3-(3,4-dihydroxyphenyl)-1-oxo-2-propen-1-yl]oxy]-1,4-dihydroxy-cyclohexanecarboxylic acid, (1R,3R,4S,5R)-3,4-bis[[3-(3,4-dihydroxyphenyl)-1-oxo-2-propen-1-yl]oxy]-1,5-dihydroxy-cyclohexanecarboxylic acid or a mixture thereof in masking the bitter taste of a consumable.

A consumable includes, for example, a food product (e.g., a beverage), a sweetener such as a natural sweetener or an artificial sweetener, a pharmaceutical composition, a dietary supplement, a nutraceutical, a dental hygienic composition and a cosmetic product. The consumable may further contain a flavoring.

In some embodiments, a consumable is a food product including, for example, but not limited to, fruits, vegetables, juices, meat products such as ham, bacon and sausage, egg products, fruit concentrates, gelatins and gelatin-like products such as jams, jellies, preserves and the like, milk products such as ice cream, sour cream and sherbet, icings, syrups including molasses, corn, wheat, rye, soybean, oat, rice and barley products, nut meats and nut products, cakes, cookies, confectioneries such as candies, gums, fruit flavored drops, and chocolates, chewing gums, mints, creams, pies and breads. In a certain embodiment, the food product is a beverage including, for example, but not limited to, coffee, tea, carbonated soft drinks, such as COKE and PEPSI, non-carbonated soft drinks and other fruit drinks, sports drinks such as GATORADE and alcoholic beverages such as beers, wines and liquors. A consumable also includes prepared packaged products, such as granulated flavor mixes, which upon reconstitution with water provide non- carbonated drinks, instant pudding mixes, instant coffee and tea, coffee whiteners, malted milk mixes, pet foods, livestock feed, tobacco, and materials for baking applications, such as powdered baking mixes for the preparation of breads, cookies, cakes, pancakes, donuts and the like. A consumable also includes diet or low-calorie food and beverages containing little or no sucrose. A preferred consumable includes carbonated beverages. Consumables further include condiments such as herbs, spices and seasonings, flavor enhancers (e.g., monosodium glutamate), dietetic sweeteners and liquid sweeteners.

In other embodiments, a consumable is a pharmaceutical composition, a dietary supplement, a nutraceutical, a dental hygienic composition or a cosmetic product. Preferred compositions are pharmaceutical compositions containing naringenin, one or more pharmaceutically acceptable excipients, and one or more active agents that exert a biological effect other than sweetness enhancement. Such active agents include pharmaceutical and biological agents that have an activity other than taste enhancement. Such active agents are well known in the art (See, e.g., The Physician's Desk Reference). Such compositions can be prepared according to procedures known in the art, for example, as described in Remington's Pharmaceutical Sciences, Mack Publishing Co., Easton, PA. In one embodiment, such an active agent includes a bronchodilator, an anorexiant, an antihistamine, a nutritional supplement, a laxative, an analgesic, an anaesthetic, an antacid, a H2-receptor antagonist, an anticholinergic, an antidiarrheal, a demulcent, an antitussive, an antinauseant, an antimicrobial, an antibacterial, an antifungal, an antiviral, an expectorant, an anti-inflammatory agent, an antipyretic and a mixture thereof. In another embodiment, the active agent is selected from the group consisting of an antipyretic and analgesic, e.g., ibuprofen, acetaminophen or aspirin, a laxative, e.g., phenolphthalein dioctyl sodium sulfosuccinate, an appetite depressant, e.g., an amphetamine, phenylpropanolamine, phenylpropanolamine hydrochloride, or caffeine, an antacid, e. g., calcium carbonate, an antiasthmatic, e. g. , theophylline, an antidiarrheal, e.g., diphenoxylate hydrochloride, an agent against flatulence, e.g., simethecon, a migraine agent, e.g., ergotamine tartrate, a psychopharmacological agent, e. g., haloperidol, a spasmolytic or sedative, e.g., phenobarbital, an aniihyperkinetic, e.g., methyldopa or methylphenidate, a tranquilizer, e.g., a benzodiazepine, hydroxyzine, meprobramate or phenothiazine, an antihistaminic, e.g., astemizol, chlorpheniramine maleate, pyridamine maleate, doxlamine succinate, brompheniramine maleate, phenyltoloxamine citrate, chlorcyclizine hydrochloride, pheniramine maleate, or phenindamine tartrate, a decongestant, e.g., phenylpropanolamine hydrochloride, phenylephrine hydrochloride, pseudoephedrine hydrochloride, pseudoephedrine sulfate, phenylpropanolamine bitartrate, or ephedrine, a beta-receptor blocker, e.g., propranolol, an agent for alcohol withdrawal, e.g., disulfuram, an antitussive, e.g., benzocaine, dextromethorphan, dextromethorphan hydrobromide, noscapine, carbetapentane citrate, and chlophedianol hydrochloride, a fluorine supplement, e.g., sodium fluoride, a local antibiotic, e.g., tetracycline or clindamycin, a corticosteroid supplement, e.g., prednisone or prednisolone; an agent against gout, e.g., colchicine or allopurinol, an antiepileptic, e.g., phenytoin sodium, an agent against dehydration, e.g., electrolyte supplements, an antiseptic, e.g., cetylpyridinium chloride, a NSAID, e.g., acetaminophen, ibuprofen, naproxen, or a salt thereof, a gastrointestinal active agent, e.g., loperamide and famotidine, an alkaloid, e.g., codeine phosphate, codeine sulfate, or morphine, a supplement for trace elements, e.g., sodium chloride, zinc chloride, calcium carbonate, magnesium oxide, and other alkali metal salts and alkali earth metal salts; a vitamin, an ion-exchange resin, e.g., cholestyramine, a cholesteroldepressant and lipid-lowering substance, an antiarrhythmic, e.g., N-acetylprocainamide and an expectorant, e.g., guaifenesin. Examples of dietary supplements or nutraceuticals include, for example, but are not limited to, an enteral nutrition product for treatment of nutritional deficit, trauma, surgery, Crohn's disease, renal disease, hypertension, obesity and the like, to promote athletic performance, muscle enhancement or general well-being or inborn errors of metabolism such as phenylketonuria, in particular, such compositions can contain one or more amino acids which have a bitter or metallic taste or aftertaste. Such amino acids include, for example, but are not limited to, an essential amino acid such as L isomers of leucine, isoleucine, histidine, lysine, methionine, phenylalanine, threonine, tryptophan, tyrosine and valine. Denial hygienic compositions are known in the art and include, for example, but not limited to, a toothpaste, a mouthwash, a plaque rinse, a dental floss, a dental pain reliever (such as ANBESOL) and the like. In one embodiment, the dental hygienic composition includes one natural sweetener. In another embodiment, the dental hygienic composition includes more than one natural sweetener, in yet another embodiment, the dental hygienic composition includes sucrose and corn syrup, or sucrose and aspartame. A cosmetic product includes, for example, but not limited to, a face cream, a lipstick, a lip gloss and the like. Other suitable cosmetic products of use in this invention include a lip balm, such as CHAPSTICK or BURT'S BEESWAX Lip Balm.

A consumable acid is an acid that is edible or suitable for human consumption at a given amount. A consumable acid includes, for example, but not limited to, acetic acid, allantoic acid, A-ketoglutaric acid, ascorbic acid, aspartic acid, benzoic acid, cetostearic acid, citramalic acid, citric acid, formic acid, fumaric acid, galacturonic acid, glucoronic acid, glutamic acid, glyceric acid, glycolic acid, hydrochloric acid, isocitric acid, lactic acid, lactarimic acid, lactoisocitric acid, malic acid, oxalacetic acid, oxalic acid, phosphoric acid, pyroglutamic acid, pviTolidinonecarboxylic acid, pyruvic acid, quinic acid, shikimic acid, succinic acid, sulphuric acid and tartaric acid. A consumable acid is a flavor compound that contributes to the sour taste of a consumable. A sour taste masking compound reduces and/or suppresses the sour taste and acid perception of a consumable acid in a consumable.

The bitter taste of a consumable is due to the presence of a flavor compound such as a bitter-tasting substance. To eliminate and/or reduce the bitter taste, a number of methods have been developed including the use of an adsorbent, the use of a clathrate compound and the addition of a sweetener (US 5,785,984). However, there remain problems such as incomplete elimination, and new problems such as changed taste and inferior flavor may arise. A bitter taste masking compound selectively reduces and/or suppresses the bitter taste and the bitterness perception of a bitter-tasting substance in a consumable.

The term "olfactory effective amount" is understood to mean the amount of a sour taste masking compound used in a consumable, wherein the sour taste masking compound reduces and/or suppresses the sour taste of the consumable.

The olfactory effective amount may vary depending on many factors including other ingredients, their relative amounts and the olfactory effect that is desired. Any amount of a sour taste masking compound that provides the desired degree of sour taste masking effect without exhibiting off-taste can be used. In the present invention the olfactory effective amount ranges from about 1 part per billion to about 1000 parts per million by weight, more preferably from about 50 parts per billion to about 100 parts per million by weight, even more preferably from about 1 to about 50 parts per million by weight. The term "ppm" is understood to mean part per million by weight.

Additional materials can also be used in conjunction with the compounds of the present invention to encapsulate and/or deliver the sour taste masking effect Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica.

The invention is described in greater detail by the following non-limiting examples.

Materials were purchased from Aldrich Chemical Company unless noted otherwise. The compounds of Formula (II), (III), (IV), (V) and (VI) are reference compounds not forming part of the claimed invention.

### Example I: Preparation of Test Samples

A series of cyclohexanecarboxylic acid solutions in the following were prepared (10 ppm in water):
1. (1S,3R,4R,5R)-3-[[3-(3,4-dihydroxyphenyl)-1-oxo-2-propen-1-yl]oxy]-1,4,5-trihydroxy-cyclohexanecarboxylic acid (Formula I);
2. (1R,3R,4S,5R)-3-[[(2E)-3-(3,4-dihydroxyphenyl)-1-oxo-2-propen-1-yl]oxy]-1,4,5-trihydroxy-cyclohexanecarboxylic acid (Formula II);
3. (1α,3R,4α,5R)-4-[[3-(3,4-dihydroxyphenyl)-1-oxo-2-propen-1-yl]oxy]-1,3,5-trihydroxy-cyclohexanecarboxylic acid (Formula III);
4. (1S,3R,4R,5R)-3,4-bis[[3-(3,4-dihydroxyphenyl)-1-oxo-2-propenyl]oxy]-1,5-dihydroxy-cyclohexanecarboxylic acid (Formula IV);
5. (1α,3R,4α,5R)-3,5-bis[[3-(3,4-dihydroxyphenyl)-1-oxo-2-propen-1-yl]oxy]-1,4-dihydroxy-cyclohexanecarboxylic acid (Formula V);
6. (1R,3R,4S,SR)-3,4-bis[[3-(3,4-dihydroxyphenyl)-1-oxo-2-propen-1-yl]oxy]-1,5-dihydroxy-cyclohexanecarboxylic acid (Formula VI); and
7. a mixture of Formula I, II, III, IV, V and VI in a weight ratio of 28.1:4.3:7.9:2.0:2.5:3.0 (51.7%).

### Example II; Sour Taste-Masking Effect

Lactic acid solution (0.09% by weight in water) and citric acid solution (0.07% by weight in water) were prepared and used, respectively, to evaluate the sour taste-masking effect of cyclohexanecarboxylic acids, which is expressed using an intensity scale of 0 to 10, where 0 = total masking, 2 = intense masking, 5 = moderate masking, 8 = weak masking and 10 = no masking. The intensity scores of cyclohexanecarboxylic acids were as follows:

| **Sample Index** | **Cyclohexanecarboxylic Acid** | **Intensity Score** |
|---|---|---|
| 1 | Formula I | 2 |
| 2 | Formula II | 4 |
| 3 | Formula III | 3 |
| 4 | Formula IV | 8 |
| 5 | Formula V | 8 |
| 6 | Formula VI | 8 |
| 7 | Mixture of Formulas (I-VI) | 5 |

Among all cyclohexanecarboxylic acids evaluated, Formula I exhibited particularly intense and lasting sour taste-masking effect. Such advantageous property is unexpected. The above sour taste-masking effect was found the same in both lactic acid and citric acid.

### Reference Example Ill: Bitter Taste-Masking Effect

Caffeine solution (0.07% by weight in water) was prepared and used to evaluate the bitter taste-masking effect of cyclohexanecarboxylic acids, which is expressed using an intensity scale of 0 to 10, where 0 = total masking, 2 = intense masking, 5 = moderate masking, 8 = weak masking and 10 = no masking. The intensity scores of cyclohexanecarboxylic acids were as follows:

| **Sample Index** | **Cyclohexanecarboxylic Acid** | **Intensity Score** |
|---|---|---|
| 1 | Formula I | 6 |
| 2 | Formula II | 7 |
| 3 | Formula III | 9 |
| 4 | Formula IV | 2 |
| 5 | Formula V | 3 |
| 6 | Formula VI | 2 |
| 7 | Mixture of Formulas (I-VI) | 8 |

Among all cyclohexanecarboxylic acids evaluated, Formula IV, Formula V and Formula VI exhibited particularly intense and lasting bitter taste-masking effect. Such advantageous properties are unexpected.

## Claims

1. A combination consisting essentially of an olfactory effective amount of (1S,3R,4R,5R)-3-[[3-(3,4-dihydroxyphenyl)-1-oxo-2-propen-1-yl]oxy]-1,4,5-trihydroxy-cyclohexanecarboxylic acid and a consumable acid, wherein the olfactory effective amount is from 1 part per billion to 1000 parts per million, and wherein (1S,3R,4R,5R)-3-[[3-(3,4-dihydroxyphenyl)-1-oxo-2-propen-1-yl]oxy]-1,4,5-trihydroxy-cyclohexanecarboxylic acid provides a sour taste-masking effect in the consumable acid.

2. The combination of claim 1, wherein the olfactory effective amount is from 50 parts per billion to 100 parts per million.

3. The combination of claim 1, wherein the olfactory effective amount is from 1 to 50 parts per million.

4. Use of an olfactory effective amount of (1S,3R,4R,SR)-3-[[3-(3,4-dihydroxyphenyl)-1-oxo-2-propen-1-yl]oxy]-1,4,5-trihydroxy-cyclohexanecarboxylic acid to mask the sour taste of a consumable, wherein the olfactory effective amount is from 1 part per billion to 1000 parts per million.

5. The use of claim 4, wherein the consumable is a food product, a sweetener, a pharmaceutical composition, a dietary supplement, a neutraceutical, a dental hygienic composition and/or a cosmetic product.

6. The use of claim 4, wherein the olfactory effective amount is from 50 parts per billion to 100 parts per million.

7. The use of claim 4, wherein the olfactory effective amount is from 1 to 50 parts per million.

## Patentansprüche

1. Kombination, im Wesentlichen bestehend aus einer olfaktorisch wirksamen Menge von (1S,3R,4R,5R)-3-[[3-(3,4-Dihydroxyphenyl)-1-oxo-2-propen-1-yl]oxy]-1,4,5-trihydroxycyclohexancarbonsäure und einer verzehrbaren Säure, wobei die olfaktorisch wirksame Menge 1 Teil pro Milliarde bis 1000 Teile pro Million beträgt und wobei (1S,3R,4R,5R)-3-[[3-(3,4-Dihydroxyphenyl)-1-oxo-2-propen-1-yl]oxy]-1,4,5-trihydroxycyclohexancarbonsäure einen den sauren Geschmack in der verzehrbaren Säure maskierenden Effekt bereitstellt.

2. Kombination nach Anspruch 1, wobei die olfaktorisch wirksame Menge 50 Teile pro Milliarde bis 100 Teile pro Million beträgt.

3. Kombination nach Anspruch 1, wobei die olfaktorisch wirksame Menge 1 bis 50 Teile pro Million beträgt.

4. Verwendung einer olfaktorisch wirksamen Menge von (1S,3R,4R,5R)-3-[[3-(3,4-Dihydroxyphenyl)-1-oxo-2-propen-1-yl]oxy]-1,4,5-trihydroxycyclohexancarbonsäure zur Maskierung des sauren Geschmacks eines Verbrauchsguts, wobei die olfaktorisch wirksame Menge 1 Teil pro Milliarde bis 1000 Teile pro Million beträgt.

5. Verbindung nach Anspruch 4, wobei es sich bei dem Verbrauchsgut um ein Lebensmittelprodukt, ein Süßungsmittel, eine pharmazeutische Zusammensetzung, eine Nahrungsergänzung, ein Nutrazeutikum, eine Zahnhygienezusammensetzung und/oder ein Kosmetikprodukt handelt.

6. Verwendung nach Anspruch 4, wobei die olfaktorisch wirksame Menge 50 Teile pro Milliarde bis 100 Teile pro Million beträgt.

7. Verwendung nach Anspruch 4, wobei die olfaktorisch wirksame Menge 1 bis 50 Teile pro Million beträgt.

## Revendications

1. Combinaison essentiellement constituée d'une quantité efficace sur le plan olfactif de l'acide (1S,3R,4R,5R)-3-[[3-(3,4-dihydroxyphényl)-1-oxo-2-propén-1-yl]oxy]-1,4,5-trihydroxy-cyclohexanecarboxylique et d'un acide consommable, la quantité efficace sur le plan olfactif étant de 1 partie par milliard à 1 000 parties par million, et l'acide (1S,3R,4R,5R)-3-[[3-(3,4-dihydroxyphényl)-1-oxo-2-propén-1-yl]oxy]-1,4,5-trihydroxy-cyclohexanecarboxylique fournissant un effet de masquage de goût aigre dans l'acide consommable.

2. Combinaison selon la revendication 1, la quantité efficace sur le plan olfactif étant de 50 parties par milliard à 100 parties par million.

3. Combinaison selon la revendication 1, la quantité efficace sur le plan olfactif étant de 1 à 50 parties par million.

4. Utilisation d'une quantité efficace sur le plan olfactif de l'acide (1S,3R,4R,5R)-3-[[3-(3,4-dihydroxyphényl)-1-oxo-2-propén-1-yl]oxy]-1,4,5-trihydroxy-cyclohexanecarboxylique pour masquer le goût aigre d'un produit consommable, la quantité efficace sur le plan olfactif étant de 1 partie par milliard à 1 000 parties par million.

5. Utilisation selon la revendication 4, le produit consommable étant un produit alimentaire, un édulcorant, une composition pharmaceutique, un supplément diététique, un nutraceutique, une composition hygiénique dentaire et/ou un produit cosmétique.

6. Utilisation selon la revendication 4, la quantité efficace sur le plan olfactif étant de 50 parties par milliard à 100 parties par million.

7. Utilisation selon la revendication 4, la quantité efficace sur le plan olfactif étant de 1 à 50 parties par million.
